# EUROPEAN PATENT APPLICATION

(11) **EP 2 541 251 A1**
(43) Date of publication of application: **02.01.2013**
(21) Application number: 10846535.2
(22) Date of filing: 26.02.2010
(51) Int. Cl.: G01N 33/579, G01N 33/551

(54) **MEASUREMENT METHOD FOR PHYSIOLOGICALLY ACTIVE SUBSTANCE OF BIOLOGICAL ORIGIN, AND REAGENT KIT FOR MEASUREMENT**

(71) Applicant: Kowa Company, Ltd., Nagoya-shi, Aichi-ken 460-8625 (JP)
(72) Inventor: YABUSAKI, Katsumi, Hamamatsu-shi Shizuoka 431-2103 (JP)
(74) Representative: Elsy, David
(86) International application number: PCT/JP2010/053125
(87) International publication number: WO 2011/104871

(57) **Abstract**

Discolosed is a technique which simplifies the adjustment of a reagent which makes the proteins contained in limulus amoebocyte lysate (LAL) attach to the surface of microparticles dispersed in a prepared drug solution, and which can improve the accuracy of the detection of a predetermined physiologically active substance or the measurement of the concentration thereof. A reagent is prepared which adsorbs the proteins contained in LAL to beads dispersed in a drug solution prepared in advance. By reacting a sample containing an endotoxin with the reagent, groups of the beads associate and rapidly form large aggregates, and measurement of the endotoxin is carried out by the detecting the formation of the aggregates. The beads are formed from an inorganic material such as alumina, kaolin, or manganese oxide.

## Description

### Technical Field

The present invention relates to, in a sample containing a physiologically active substance of biological origin which has the property of forming a gel by a reaction between endotoxin, β-D-glucan or the like with a limulus amoebocyte lysate (LAL), a method of detecting the physiologically active substance or measuring the concentration of the physiologically active substance, and a reagent kit to be used for the detection or concentration measurement.

### Background Art

Endotoxin is a lipopolysaccharide present in a cell wall of a Gram-negative bacterium and is the most typical pyrogen. If a transfusion, a medicine for injection, blood or the like contaminated with the endotoxin enters into a human body, the endotoxin may induce severe side effects such as fever and shock. Therefore, it is required to manage the above medicines so that they are not contaminated with endotoxin.

By the way, a hemocyte extract of limulus (hereinafter, also referred to as "limulus amoebocyte lysate (LAL)") contains serine protease activated by endotoxin. When LAL reacts with endotoxin, a coagulogen present in LAL is hydrolyzed into a coagulin by an enzyme cascade by the serine protease activated according to the amount of endotoxin, and the coagulin is associated to form an insoluble gel. With the use of this property of LAL, it is possible to detect endotoxin with high sensitivity.

Furthermore, β-D-glucan is a polysaccharide that constitutes a cell membrane characteristic of a fungus. Measurement of β-D-glucan is effective, for example, for screening of infectious diseases due to a variety of fungi including not only fungi that are frequently observed in general clinical practices, such as Candida, Spergillus, and Cryptococcus, but also rare fungi. Also in the measurement of β-D-glucan, by using the property of the limulus amoebocyte lysate to coagulate (coagulate to form a gel) by β-D-glucan, the β-D-glucan can be detected with a high sensitivity.

A method for detection or concentration measurement of a physiologically active substance of biological origin (hereinafter, also referred to as "predetermined physiologically active substance") such as endotoxin and β-D-glucan, which can be detected by a limulus amoebocyte lysate (hereinafter, also simply referred to as "measurement of the predetermined physiologically active substance") includes a semi-quantitative gelation method including: allowing a blended mixture obtained by blending a sample with LAL to stand; inverting the vessel after a lapse of a predetermined time; and determining whether or not the sample is gelled based on the presence or absence of dipping of the sample so as to examine whether or not the sample contains the predetermined physiologically active substance of a certain concentration or more. Other examples of a highly sensitive method of quantifying the predetermined physiologically active substance include a turbidimetric method including analyzing a sample by measuring over time the turbidity of the sample caused by gel formation by a reaction between LAL and the predetermined physiologically active substance (including a stirring turbidimetric method of measuring the turbiditywhile stirring a sample) and a colorimetric method using a synthetic substrate that is hydrolyzed by an enzyme cascade to develop a color.

Among the above quantification methods, the turbidimetric method may require a very long reaction time until gel formation is detected because the amount of an enzyme activated by an action of a low concentration of a predetermined physiologically active substance is small. On the other hand, the colorimetric method has a disadvantage that the method is easily affected by the turbidity (such as blood lipid) of a sample and a pigment mixed (such as hemoglobin caused by hemolysis). Therefore, the above turbidimetric method and colorimetric method are not necessarily the best methods for measurement of the predetermined physiologically active substance in blood of a patient in emergency or for measurement of the predetermined physiologically active substance in a dialysis solution or blood in artificial dialysis.

Further, it has been proposed a laser light scattering particle counting method, which involves measuring formation of gel microparticles at an aggregation early stage by: forming gel microparticles rapidly by stirring a sample obtained by blending LAL and the predetermined physiologically active substance; and analyzing the sizes and number of the gel microparticles based on the intensity of laser light scattered by the gel microparticles. It has been reported that the method can shorten the measurement time to about one-third compared with the turbidimetric method and can reduce the effects of the turbidity or color of the sample.

However, even if the above laser light scattering particle counting method is used, it takes a time of 40 to 50 minutes to measure a low concentration of the predetermined physiologically active substance. In the laser light scattering particle counting method, the optical system is complex because particles in a minute space are observed, resulting in a disadvantage that measurement of many analytes is difficult.

On the other hand, there is proposed a measurement method of a predetermined physiologically active substance including: preparing a reagent in which proteins in LAL are bound to the surfaces of microparticles such as polystyrene latex which are previously dispersed in a drug solution; and blending the reagent with a sample containing endotoxin. According to this, the predetermined physiologically active substance is acted on the proteins on the microparticles to allow the microparticles to be associated with each other so that a larger aggregate can be generated rapidly. Then, the detection or concentration measurement of endotoxin can be performed in a very short time by optically measuring the generation of the aggregates.

However, in the measurement method using the microparticles of polystyrene latex, it is difficult to perform a dry-heat sterilization treatment at high temperatures. Accordingly, the reagent may be contaminated by endotoxin during the preparation of the reagent. In addition, in order to bind the proteins in LAL to the microparticles of polystyrene latex, it is necessary to immobilize functional groups such as carboxyl groups on the surfaces of the microparticles of polystyrene latex, and the preparation of the reagent goes through multiple processes, which is disadvantageous. This contributes to a risk of contamination of the reagent by endotoxin.

### Citation List

### Patent Literatures

Patent Literature 1: Japanese Patent No. 2667695
Patent Literature 2: Japanese Patent Application Laid-Open (JP-A) No. 2009-150723
Patent Literature 3: JP-A No. 2004-061314
Patent Literature 4: JP-A No. 10-293129
Patent Literature 5: International Publication No . 2008/038329

### Non-Patent Literature

Non-Patent Literature 1: Satoshi Fukuzaki, Hiromi Urano and Kazuya Nagata "Adsorption of bovine serum albumin onto metal oxide surfaces", Journal of Fermentation and Bioengineering Volume 81, Issue 2, 1996, Pages 163-167
Non-Patent Literature 2: Satoshi Fukuzaki, Hiromi Urano, "surface charge characteristics of oxide and adsorption behavior of protein", JOURNAL OF ANTIBACTERIAL AND ANTIFUNGAL AGENTS, JAPAN, Volume 27, No. 1, 1999, Pages 33-40

### Summary of Invention

### Technical Problem

The present invention has been made in consideration of the above problems. An obj ect of the present invention is to provide a technology which can simply prepare a reagent in which proteins in LAL are bound to the surfaces of microparticles which are previously dispersed in a drug solution and improve the accuracy of detection or concentration measurement of the predetermined physiologically active substance.

### Solution to Problem

In the method of measuring a predetermined physiologically active substance according to the present invention, a reagent in which proteins in LAL are attached to microparticles dispersed in a previously prepared drug solution is produced. Then, a sample containing a predetermined physiologically active substance is acted on the reagent to allow the microparticles to be associated with each other so that a larger aggregate can be generated rapidly. The predetermined physiologically active substance is measured by detecting the formation of the aggregates. The most distinctive feature is that the above microparticles are formed of an inorganic material.

More particularly, there is provided a measurement method for a physiologically active substance of biological origin including: allowing a predetermined protein contained in a limulus amoebocyte lysate to be attached to the surfaces of microparticles capable of being dispersed in a reagent; blending the reagent containing the dispersed microparticles to which the proteins are attached with a sample; and detecting formation of a gel in a blended liquid of the reagent and the sample to detect the physiologically active substance of biological origin present in the sample or measuring the degree of formation of the gel to measure the concentration of the physiologically active substance; wherein the microparticles are formed from an inorganic material.

Here, it has been revealed that when the predetermined physiologically active substance is acted on LAL in a state in which proteins contained in LAL (limulus amoebocyte lysate) are attached onto microparticles, a larger aggregate is generated rapidly as compared with the case where the predetermined physiologically active substance is acted on an LAL simple substance as described above. This is because the proteins on microparticles are hydrolyzed into coagulins by an enzyme cascade caused by an act of the predetermined physiologically active substance, which allows the microparticles to be associated with each other.

The present invention utilizes the phenomenon. The microparticles are associated with each other to generate larger aggregates rapidly, resulting in facilitation of the formation of the gel in the blended liquid of LAL and a sample. According to this, it is possible to carry out detection or concentration measurement of predetermined physiologically active substances more rapidly and simply.

Note that, the above predetermined protein has a property to react with predetermined physiologically active substances to form a gel and includes at least a coagulogen. Further, the protein may contain other proteins such as serine protease. In the present invention, gel formation means a phenomenon which includes at least one of formation of an aggregate by association of the microparticles as described above, formation of gel particles by association of a coagulin which is not attached to the microparticles, and gel formation of a blended liquid of LAL and a sample.

In the present invention, the degree of gel formation means an increase in the size or number of an aggregate formed by association of the microparticles as described above, and a gel particle formed by association of the coagulin which is not bound or attached to the microparticles. Alternatively, the degree means changes in physical and chemical properties (e.g., turbidity) of the blended liquid caused by gel formation of the blended liquid of the LAL reagent and the sample.

The case where microparticles of resin such as polystyrene latex are used as the above microparticles will be considered herein. In this case, the production process of microparticles does not include a process of being exposed to high heat (when exposed to high heat, the resin is denatured). Therefore, it is difficult to ensure that the microparticles themselves are not contaminated by the predetermined physiologically active substance in an early stage. Further, the proteins in LAL such as coagulogens are relatively hard to be attached to the above resin microparticles. Thus, a process of immobilizing functional groups such as carboxyl groups on microparticles to be activated is needed. This results in complication of the preparation process of the reagent. Further, this leads to an increase in contamination of the reagent by the predetermined physiologically active substance in the preparation process.

On the other hand, in the present invention, the microparticles are formed from an inorganic material. Thus, as compared with the case where microparticles are formed from an organic material such as resin, the heat resistance of the microparticles themselves is improved. Accordingly, the dry-heat sterilization treatment can be performed in the preparation process of the reagent. As a result, it is possible to suppress the contamination of the reagent by the predetermined physiologically active substance in the preparation process. Further, as compared with the case where microparticles are formed from an organic material such as resin, it is possible to improve the adsorptive property to the microparticles of the proteins. Therefore, the proteins can be electrostatically attached to the surfaces of the microparticles without immobilizing functional groups such as carboxyl groups on the microparticles. Thus, the preparation process of the reagent can be significantly simplified. This allows for reduction in contamination of the reagent by the predetermined physiologically active substance in the preparation process. Examples of the inorganic material may include metal oxides and minerals.

In the present invention, the microparticles may be formed from any of alumina, titania, kaoline, and manganese oxide. When microparticles are formed from these materials, the adsorptive property of the microparticles to the proteins in LAL can be more reliably improved, and light scattering property or light absorption property of the microparticles can be improved. Thus, in the turbidimetric method, it is possible to more clearly detect changes in the absorbance of a blended liquid of a sample and an LAL reagent. Further, in the laser light-scattering method, it is possible to more clearly detect a peak of the intensity of the scattered light from the blended liquid. As a result, the predetermined physiologically active substance can be measured with higher accuracy.

Further, when microparticles are formed from the above materials, mechanical strength sufficient as microparticles can be obtained. Thus, when a sample and a reagent containing the microparticles are blended and stirred, it is possible to inhibit changes in physical and optical properties of the blended liquid caused by the crash of microparticles due to mechanical load.

Here, the predetermined protein may be coagulogen purified from limulus amoebocyte lysate.

As described above, in the aggregation reaction when reacting a predetermined physiologically active substance with LAL, the coagulogen is hydrolyzed into coagulin by an enzyme cascade in the LAL, which allows microparticles to be associated with each other. Therefore, when only the coagulogen extracted by previously purifying proteins in LAL is attached to the microparticles, it is possible to allow microparticles to be associated with each other more efficiently and form aggregates more rapidly.

In the present invention, the reagent containing the dispersed microparticles to which the predetermined protein is attached may be mixed with limulus amoebocyte lysate, and the reagent and the sample may be blended simultaneously with or after the mixing.

According to this, the proteins in LAL can be further supplied to the reagent containing the dispersed microparticles to which the proteins are bound or attached. Thus, when mixed with the sample containing the predetermined physiologically active substance, the coagulogen attached onto the microparticles and the coagulogen mixed in LAL can be hydrolyzed into coagulins by more reliably acting the predetermined physiologically active substance on serine protease in the reagent. As a result, it is possible to more accurately associate the coagulin attached to the microparticles with each other or to more accurately associate the coagulin attached to the microparticles with the coagulin in LAL mixed, resulting in promoting formation of an aggregate mainly containing the microparticles. This allows for the measurement of the predetermined physiologically active substance in a shorter time.

This is advantageous in the case where only the coagulogen extracted by previously purifying proteins in LAL is bound or attached to the microparticles. That is, even in such a case, when the reagent is blended with the sample containing the predetermined physiologically active substance, the predetermined physiologically active substance can act on the serine protease more accurately.

Note that, in the above process, the reagent containing the dispersed microparticles to which the predetermined protein is attached is further mixed with the limulus amoebocyte lysate, and then the mixture is blended with the sample containing the predetermined physiologically active substance. The blending may be performed after or simultaneously with the mixing with the lysate. However, in order to uniformly disperse the coagulogen and serine protease supplemented in the reagent, blending with the sample containing the predetermined physiologically active substance is desirably performed after a lapse of a predetermined time from mixing of the reagent with the lysate.

The present invention may provide a reagent kit for measuring a physiologically active substance of biological origin which is prepared by allowing a predetermined protein contained in a limulus amoebocyte lysate to be attached to the surfaces of microparticles capable of being dispersed in a reagent, where the microparticles are formed from an inorganic material.

In that case, the microparticles may be formed from metal oxides or minerals. The microparticles may be formed from any of materials of alumina, titania, kaoline or manganese oxide. Further, the predetermined protein may be a coagulogen purified from the limulus amoebocyte lysate.

Note that the above solutions to the problems of the present invention can be used in combination as much as possible.

### Advantageous Effects of Invention

In the present invention, it is possible to more easily prepare a reagent in which proteins in LAL are bound to the surfaces of microparticles which are previously dispersed in a drug solution. It is possible to improve the accuracy of the detection or concentration measurement of the predetermined physiologically active substance.

### Brief Description of Drawings

Fig. 1 is a view explaining an aggregation mechanism of LAL-bound beads in the example of the present invention.
Fig. 2 is a graph showing a relation between the endotoxin concentration and detection time in both cases of using or not using the LAL-bound bead reagent according to Production example 1 in the endotoxin concentration measurement by a stirring turbidimetric method.
Fig. 3 is a graph showing temporal changes in the detected number of particles in both cases of using or not using the LAL-bound bead reagent according to Production example 2 in the endotoxin concentration measurement by a laser light scattering particle counting method.
Fig. 4 is a graph showing a relation between the endotoxin concentration and detection time in both cases of using or not using the LAL-boundbead reagent according to Production example 2 in the endotoxin concentration measurement by the laser light scattering particle counting method.
Fig. 5 is a graph showing a relation between the endotoxin concentration and detection time in both cases of using or not using the LAL-bound bead reagent prepared by using titania particles.
Fig. 6 is a graph showing the absorbance of each of the materials of beads 1 in Example 4 when irradiated with light having a wavelength of 430 nm.
Fig. 7 is a graph showing temporal changes in the light transmittance in both cases of using or not using the LAL-bound bead reagent prepared by using each of alumina, kaoline, and manganese oxide as materials of the beads in the endotoxin concentration measurement by the stirring turbidimetric method.
Fig. 8 is a graph showing temporal changes in the light transmittance in both cases of using or not using the LAL-bound bead reagent prepared by using each of alumina and silica as materials of the beads in the endotoxin concentration measurement by the stirring turbidimetric method.
Fig. 9 is a graph showing a relation between the light transmittance and stirring time when hydroxyapatite is used as a material of the beads.
Fig. 10 is an outline view for explaining a process that LAL forms a gel in the presence of endotoxin or β-D-glucan and the detection method thereof.

### Description of Embodiments

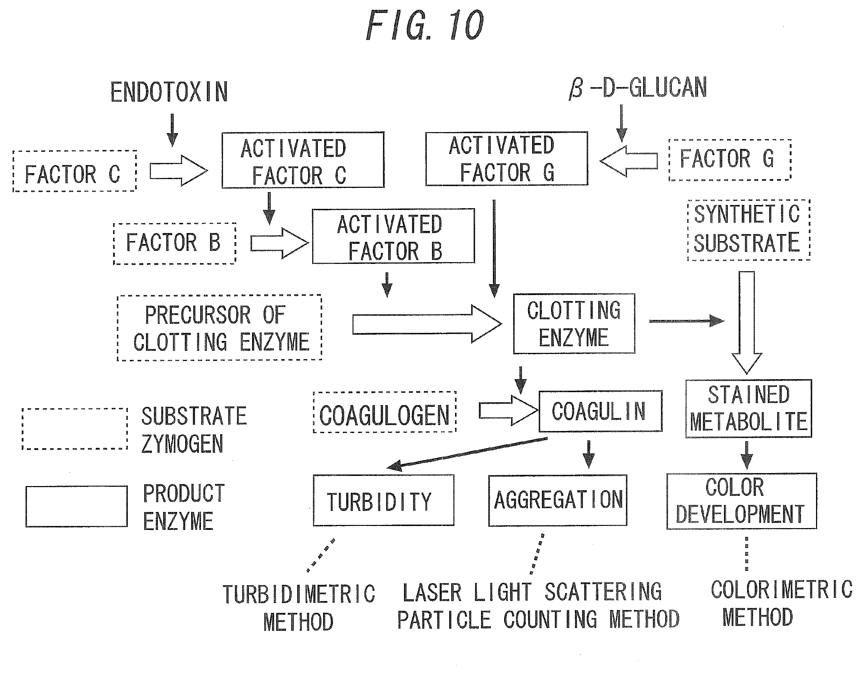

The process of forming a gel by a reaction between LAL and endotoxin has been studied well. As illustrated in Fig. 10, when endotoxin is bound to serine protease, i.e., factor C in LAL, the factor C becomes activated factor C. The activated factor C hydrolyzes and activates another serine protease, i.e., factor B in LAL, and then the factor B is activated to become activated factor B. The activated factor B immediately hydrolyzes precursor of clotting enzyme in LAL to form clotting enzyme, and further the clotting enzyme hydrolyzes coagulogen in LAL to generate coagulin. Thus, the generated coagulins are then associated with each other to further form an insoluble gel, and the whole LAL is involved in the formation to turn into a gel.

In addition, similarly, when β-D-glucan is bound to factor G in LAL, the factor G is activated to become activated factor G. The activated factor G hydrolyzes precursor of clotting enzyme in LAL to produce clotting enzyme. As a result, as with the reaction between endotoxin and LAL, coagulins are generated, and the generated coagulins are associated with each other to further generate an insoluble gel.

The series of reactions as described above are similar to the process of forming fibrin gel via serine proteases such as Christmas factor or thrombin present in mammals. Such enzyme cascade reactions have a very strong amplification effect because even a very small amount of activation factor activates the subsequent cascade in a chain reaction. Therefore, if a predetermined physiologically active substance such as endotoxin or β-D-glucan is measured using LAL, it is possible to detect a very small amount (sub-pg/mL order) of the predetermined physiologically active substance. Hereinafter, taking endotoxin (the predetermined physiologically active substance) as an example, the description will be continued. The present invention can be applied to not only endotoxin but also other predetermined physiologically active substances including β-D-glucan.

Examples of a measurement method which can quantify the endotoxin include the turbidimetric method (including the stirring turbidimetric method) and the laser light scattering particle counting method, as described above. As shown in Fig. 10, insuchmeasurement methods, measurement can be performed with high sensitivity by detecting association products of coagulin formed by the enzyme cascade reactions in LAL as the turbidity of a sample in the former method or as gel microparticles formed in the system in the latter method.

However, in the turbidimetric method, each coagulin is a microparticle (nanometer order). Therefore, even if the particles are progressively associated with each other, the particles cannot be detected as the turbidity until the product grows to a size which can be detected optically. Meanwhile, in the turbidimetric method, in general, a sample is allowed to stand to form a gel in the whole system. Accordingly, the coagulin collision probability is low, and the association rate is not always high. Therefore, in the turbidimetric method, there is a disadvantage that it takes a very long time before detection or concentration measurement of endotoxin can be achieved.

On the other hand, in the laser light scattering particle counting method, gel microparticles formed in the system are directly measured, and hence the method is more sensitive than the turbidimetric method. In addition, gel formation can be detected in a short period of time compared with the turbidimetric method because in general, a sample containing LAL and an analyte is forcibly stirred. However, in the laser light scattering particle counting method, the optical system is complex because particles in a minute space are observed, resulting in a disadvantage that measurement with a simple device is difficult.

In this embodiment, coagulogen is preliminarily bound to the surfaces of microparticles formed from an inorganic material with a size which can be detected by the turbidimetric method or the laser light scattering particle counting method or is slightly smaller than a detection limit size. Then, a sample containing endotoxin is blended in a reagent prepared by mixing the particles with an LAL reagent. Thus, a coagulin formed in LAL is associated with a coagulin formed on the microparticles, or coagulins formed on the microparticles are associated with each other, to form an aggregate with a size which can be detected by the turbidimetric method or the laser light scattering particle counting method in short time. As a result, endotoxin can be more rapidly measured by the turbidimetric method or the laser light scattering particle counting method.

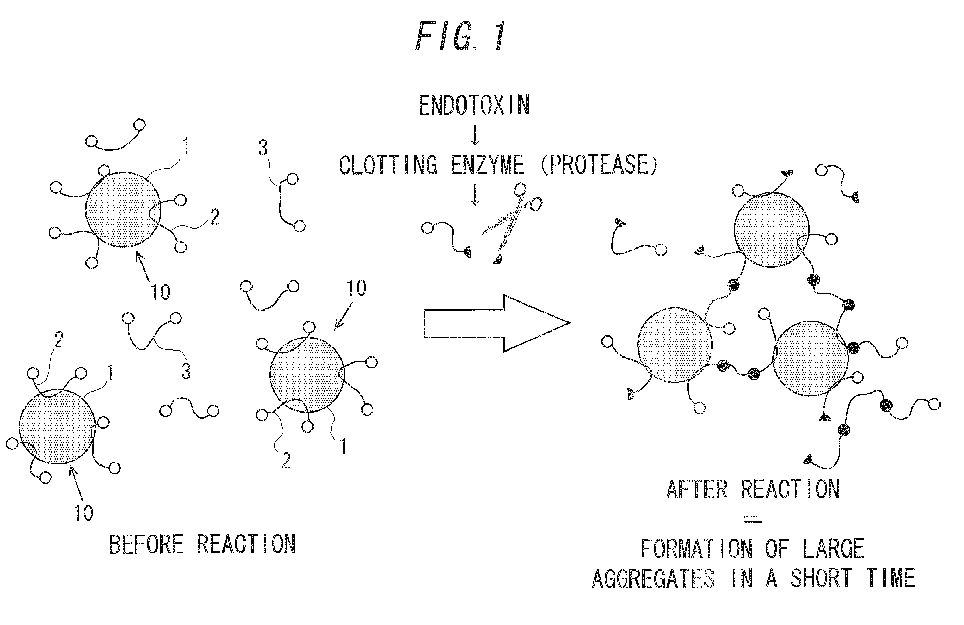

Fig. 1 shows an aggregation mechanism of microparticles according to this example. In Fig. 1, the drawing on the left side of the central arrow is a schematic view of the reagent before the reaction with endotoxin. The drawing on the right side of the arrow is a schematic view of the reagent after the reaction with endotoxin.

As shown in Fig. 1, before the reaction, bead 1, which is a microparticle in this example, coagulogen 2 which is bound to the surface of the microparticle, and coagulogen 3 which is not bound to the surface of the microparticle are dispersed. Note that, hereinafter, as a matter of convenience, the microparticle obtained by binding the coagulogen 2 to the bead 1 is referred to as LAL-bound bead 10.

The conditions to be satisfied by the material to be used for the bead 1 will be considered herein. The material to be used for the bead 1 is desirably one the bead 1 itself made from which can be dry-heat sterilized. Inotherwords, it is desired to use not an organic material such as resin, but an inorganic material. Thus, at the formation stage of the bead 1 or the preparation stage of the LAL-bound bead 10, it is possible to inactivate endotoxin by heat by dry-heat sterilization. Accordingly, a risk of contamination of the LAL-bound bead 10 by endotoxin can be reduced.

The material to be used for the bead 1 is required to have high adsorptive property to the proteins. Note that Non-Patent Literature 1 discusses the amount of adsorption of bovine serum albumin to silica, titania or alumina from the viewpoint of an isoelectric point of proteins and a zero charge point of metal oxide. Further, Non-Patent Literature 2 shows that the zero charge point of the metal oxide may largely vary depending on the kind of a buffer present in a solution. Here, a relation between the isoelectric point of the proteins and the zero charge point of the bead 1 determines whether the material has high adsorptive property to the proteins or not. That is, it is necessary to have a difference in potential between the bead 1 and proteins to allow the proteins to be attached to the bead 1. For example, there is a report in which the isoelectric point, i.e. a pH to electrically neutralize coagulogens, is about 10. Accordingly, examples of a material having a difference in potential between the coagulogens includes titania (pH 6.1) and zirconia (pH 7.3). As described in Non-Patent Literature 2, the zero charge point of alumina is high at the alkaline side (pH 9.0), however, it is easily affected by the buffer contained in the solution, and the zero charge point is largely shifted to the acidity side. Thus, even alumina can adequately be attached by coagulogens in such as a limulus reagent which contain a buffer.

The isoelectric point of the coagulogens is about 10, which is very high at the alkaline side. Thus, for example, in the neutral environment around pH 7 (at the time of preparing and using the reagent, within pH 6 to 8 in many cases), it is considered that coagulogens are charged to the positive side. Therefore, in order to attach the proteins powerfully, it is more desirable to use a material having a zero charge point such that the bead 1 are charged to the negative side in the neutral environment (pH 7). At this time, a material having a zero charge point that is charged to the negative side may be used or a material that is charged to the negative side in the presence of an appropriate buffer may be used.

Further, the material of the bead 1 is required to have more than a certain level of strength of the turbidity when the aggregation is generated. In this example, the aggregation of the bead 1 based on the reaction of the proteins in LAL and endotoxin is measured by an optical means, such as the turbidimetric method or the laser light scattering particle counting method. In other words, the aggregation of the bead 1 are measured by utilizing changes in the intensity of the transmitted or scattered light due to the blended liquid containing the dispersed bead 1. Therefore, as the material of the bead 1, it is desired to use a white material which easily scatters light, a black material which easily absorbs light or a specific colored material.

In addition, as the material of the bead 1 which is advantageous to measure endotoxin in this example, it is desired to use a material which is not dissolved inwaterduringuse. From the viewpoint of easy stirring, it is desired to use a material whose specific gravity is close to water and is not very large. Further, the particle diameter should not be changed by stirring, and thus it is desired to use a material having a mechanical strength enough to endure the stirring process.

From the above requirements, among inorganic substances, metal oxide particles insoluble in water in neutral or mineral particles are desired as the material of the bead 1. Examples of the metal oxide particles include silica, titania, zirconia, alumina, ceria, manganese oxide, ferrite, composites or mixtures thereof. In addition, magnesia (MgO), calcia (CaO), alumina (Al₂O₃), alumina-titania (Al₂O₃-TiO₂), porcelainite (3Al₂O₃-2SiO₂), spinel (Al₂O₃-MgO) , zirconia (ZrO₂), chromia (Cr₂O₃), yttria (Y₂O₃), ceria (CeO₂), barium titanate (BaTiO₃), hafnia (HfO₂) or the like may be used as a material of the bead 1 according to this example.

In addition, the particle size of the bead 1 of this embodiment needs to be uniform. Particularly, when endotoxin is measured by the laser light scattering particle counting method, the uniformity of the particle size is an important factor. When endotoxin is blended with the reagent containing the LAL-bound bead 10, the progression of the aggregation is varied if the particle size of the bead 1 is not uniform. As a result, changes in turbidity and the number of particles become slow. This may lead to disadvantages such as a decrease in measurement accuracy and a decrease in reproducibility of measurement. Examples of a material which is currently available as particles having a sufficiently uniform particle size include titania, alumina, and manganese oxide.

When allowing the coagulogens 2 to be attached to the bead 1, coagulogens may be attached to the bead 1 without screening the proteins contained in LAL. Alternatively, the coagulogens in LAL may be purified to allow them to be attached. When the method of allowing the coagulogens to be attached to the microparticle without screening the proteins contained in LAL was used, proteins other than the coagulogens 2 may be attached to the bead 1. However, since the process of purifying the coagulogens in LAL can be omitted, the cost for preparing and purifying the reagent can be reduced. In that case, more proteins (not shown) contained in LAL other than the coagulogens 2 and 3 are present in the reagent on and around the bead 1 in Fig. 1.

Subsequently, the actual detection or concentration measurement of endotoxin with the LAL-bound bead reagent in this embodiment will be described while comparing to the case where polystyrene latex was used as a material of the bead 1.

### <Comparative Production Example 1> (Case where polystyrene latex was used)

As Comparative production example, there will be described a production example of an LAL-bound bead reagent obtained by binding polystyrene latex particles each having a carboxyl group on the bead surface to the LAL reagent under an environment where an irreversible enzyme inhibitor is added. Specifically, as the bead 1 having a carboxyl group on the surface, Polybead Calboxylate Microsphere (hereinafter, abbreviated as "PCM") having a particle diameter of 0.45 µm or 1.0 µm, manufactured by Polysciences, Inc. was used.

As the LAL reagent, a reagent for detecting endotoxin manufactured by Wako Pure Chemical Industries, Ltd., Limulus HS-T Single Test Wako (hereinafter, abbreviated as "LAL reagent" or "limulus reagent") was used. 1.0 mL of PCM was placed in a centrifugation tube with a maximum volume of 2.0 mL, and 0.1M carbonate buffer (pH 9.6) was added up to the maximum volume. Then, the whole was mixed well and centrifuged using a desktop centrifuge at 12,500 rpm for 5 minutes to precipitate PCM, and the supernatant was removed. A series of procedures including blending with the carbonate buffer, centrifugation, and removal of the supernatant were further repeated twice. Subsequently, 0.02 M phosphate buffer (pH 4.5) was added to the precipitated PCM up to the maximum volume, and the whole was blended. Then, the procedures of centrifugation and removal of the supernatant were performed three times in total in the same manner as above.

Subsequently, the PCM after centrifugation and removal of the supernatant was heat-treated in an autoclave at 121°C for 20 minutes. Accordingly, even if the PCM is contaminated by endotoxin at this time, most of the endotoxin can be inactivated. Thereafter, the PCM was washed with a buffer.

Then, 0.75 mL of phosphate buffer was added to the resultant PCM precipitates, and 0.75 mL of a 2.0% aqueous solution of carbodiimide (water-soluble carbodiimide, manufactured by Dojindo Laboratories) was further added thereto, followed by stirring by turning upside down at room temperature for 3 hours to activate carboxyl groups on the PCM.

After the reaction, the PCM was centrifuged to remove the supernatant and washed with the phosphate buffer three times in the same manner as above, and the final PCM precipitates were suspended in 0.5 mL of 0.2 M borate buffer (pH 8.5). Subsequently, the LAL reagent was dissolved in 0.25 mL of borate buffer to prepare two LAL reagent solutions, and the solutions were mixed with the PCM suspension to a volume of 1.0 mL. Further, a serine protease inhibitor, phenylmethanesulfonyl fluoride (PMSF), was added thereto so that the final concentration was 4 mM. The mixed solution was allowed to react at room temperature for 4 hours or more so that the activated carboxyl groups on the PCM in the mixed solution were bound to amino groups of each protein in LAL via amide bonds.

Thereafter, the mixed solution was centrifuged to remove the supernatant, and the residue was resuspended in 1.2 mL of borate buffer. Further, 50 µL of 0.25 M 2-aminoethanol/borate buffer was added to the solution. The solution was stirred by turning upside down at room temperature for 30 minutes to consume unreacted activated carboxyl groups on the PCM with aminoethanol. Further, the PCM suspension was centrifuged to obtain PCM precipitates, and the precipitates were washed three times in total by a series of procedures including resuspension in physiological saline for injection (manufactured by Otsuka Pharmaceutical Co., Ltd.), centrifugation, and removal of the supernatant, to obtain an LAL-bound bead reagent. Finally, the LAL-bound bead reagent was prepared to an optimal concentration by adding a preservative and a surfactant.

As described above, when polystyrene latex is used as a material of the bead 1, the LAL-bound bead reagent is produced using so many processes. It may take two days to obtain the LAL-bound bead reagent.

On the other hand, in the LAL-bound bead reagent according to this embodiment, metallic oxide particles of an inorganic substance, specifically, microparticles of alumina are used as the bead 1. Hereinafter, a method of preparing an LAL-bound bead reagent when microparticles of alumina are used as the bead 1 will be described.

The bead 1 of alumina may be formed by, for example, spraying atomized aluminum aerosol on flames to oxidize particles of the aerosol. As is clear from this, the bead 1 of alumina is usually formed at a high temperature of 1000°C or more. Thus, it can be ensured that the microparticles are not contaminated by endotoxin at least in the stage of forming the bead 1.

As for alumina, when the particle size is large (0.3 to 0.4 µm as a primary particle size), it is suitable for a procedure of measuring the light transmittance (absorbance) since the particle itself is a strong light scattering body. When the particle size is small (0.1 µm or less as a primary particle size), it is suitable to use in light scattering counting methods (the laser light scattering particle counting method, the Rayleigh scattering method, etc.) since the particle itself does not produce a very strong scattering.

### <Production Example 1> (Production of LAL-bound bead for light transmittance measurement)

Spherical alumina particles (ASFP-20, manufactured by DENKI KAGAKU KOGYO KABUSHIKI KAISHA, average particle diameter: 0.3 µm) were subjected to a dry heat treatment (at 250°C for 3 hours) and the contaminated endotoxin was inactivated by heat. The spherical alumina particles can be sterilized by hot air at high temperatures and thus the endotoxin can be completely inactivated at this stage.

Subsequently, the spherical alumina particles were dispersed in distilled water for injection at a concentration of 20 mg/mL. The dispersion liquid was centrifuged at 1000 rpm for 3 minutes, and clumps of the particles or large-sized particles were centrifuged and removed. Then, the dispersion liquid of spherical alumina particles was added in an amount of 300 µL per bottle of a limulus reagent (HS-T, manufactured by Wako Pure Chemical Industries, Ltd.,) to dissolve the limulus reagent. The resulting solution was stirred and blended with a vortex mixer, followed by heat-treatment at 60°C for 20 minutes using an aluminium block heater to allow proteins in the limulus reagent to be attached and immobilized onto spherical alumina particles.

Note that, as described above, the spherical alumina particles have strong adsorptivity to proteins such as coagulogen, and further, the proteins are more easily attached and immobilized by heating. Therefore, in this embodiment, the immobilization of the proteins onto the spherical alumina particles can be realized by a simpler process. Further, the heat treatment allows serine protease in the limulus reagent to be completely deactivated. Therefore, when the spherical alumina particles are used, it is unnecessary to add a serine protease inhibitor such as PMSF. After the heat-treatment, a preservative (sodium azide) and a surfactant (TritonX-100) were added at 0.05% and diluted with distilled water for injection so as to have a total amount of 3.0 mL.

Thus, the production process of the LAL-bound bead reagent could be further simplified by using the microparticles of alumina which was an inorganic substance and a metal oxide as the material of the bead 1 (total time: 30 minutes to 1 hour). This enables the risk of causing contamination by endotoxin to be reduced during preparing the LAL-bound bead reagent. Since the bead 1 is an inorganic substance (metal oxides), endotoxin can be completely inactivated by the hot-heat treatment of high temperatures.

Further, when the microparticles of polystyrene latex were used as the material of the bead 1, carboxyl groups immobilized on the microparticles were bound to proteins in LAL via amide bonds. In this case, the cross-linking between the proteins is easily caused. As a result, a condensate of the bead 1 is easily generated and thus it may be difficult to uniformly maintain the dispersibility of the bead 1.

On the other hand, when the spherical alumina particles are used as the material, proteins are attached and immobilized onto the alumina surfaces by adsorptivity to the proteins in the microparticles of alumina. Therefore, it is possible to form a state in which the whole surface of the bead 1 is covered with the proteins without cross-linking the beads 1 to each other by adjusting the concentration of the beads 1 and the concentration of the proteins. Then, a casual aggregation of the bead 1 can be inhibited, and the uniformity of the distribution in the LAL-bound bead reagent can be improved. In this regard, in the present Production example, the heat-treatment was performed at 60°C for 20 minutes using the aluminium block heater to allow proteins in the limulus reagent to be attached and immobilized onto spherical alumina particles. However, the heat treatment is not necessarily needed because the spherical alumina particles have a sufficiently high adsorptive property to the proteins.

### <Production Example 2> (Production of LAL-bound bead for laser light scattering particle counting method)

Alumina particles (ALuC, manufactured by Aerosil Co., Ltd., primary particle diameter: 0.03 µm) were subjected to a dry heat treatment (at 250°C for 3 hours) and the contaminated endotoxin was inactivated by heat. Subsequently, the particles were dispersed in distilled water for injection at a concentration of 10 mg/mL. The dispersion liquidwas centrifuged at 3000 rpm for 1 minute, and clumps of the particles or large-sized particles were centrifuged and removed. Then, the dispersion liquid of microparticles which was 2-fold diluted with distilled water for injection was added in an amount of 100 µL per bottle of a limulus reagent (HS-T, manufactured by Wako Pure Chemical Industries, Ltd.,) to dissolve the limulus reagent.

The mixture was stirred and blended in a vortex mixer. Thereafter, the resulting mixture was heat-treated in an Aluminum Block Heater at 60°C for 20 minutes to allow proteins in the limulus reagent to be attached and immobilized onto alumina particles. Further, the serine protease in the limulus reagent was completely deactivated by the heat treatment. After the heat-treatment, a preservative (sodium azide) and a surfactant (TritonX-100) were added at 0.05% and diluted with distilled water for injection so as to have a total amount of 1.5 mL.

### <Production Example 3> (Endotoxin-free glass vessel)

In the light transmittance measuring method, a special-purpose glass vessel having φ6 mm and length of 50 mm equipped with a stainless steel stirring bar for stirring a sample (φ0.75 mm and 3.5 mm in length) was used. On the other hand, in the laser light scattering particle counting method, a special-purpose glass vessel having φ7 mm and length of 50 mmm equipped with a stainless steel stirring bar for stirring a sample (φ1 mm and 5 mm in length) was used. An opening of the vessel was covered with aluminum foil, and further, each 20 of the vessels were packaged with aluminum foil and contained in a dried and heat-treated iron can, and heat-treated at 250°C for three hours so as to inactivate endotoxin.

Subsequently, the measurement of endotoxin using the LAL-bound bead reagent prepared by using the spherical alumina particles produced in the above manner will be described.

### <Example 1> (Light transmittance measurement)

50 µL of endotoxin dilution series (prepared by diluting with distilled water for injection so as to be 2, 0.2, 0.02, 0.002, and 0.0002 EU/mL) was poured into the measuring glass vessel (φ6 mm) produced in Production example 3. Then, a limulus reagent (ES-II, manufactured by Wako Pure Chemical Industries, Ltd.) was dissolved in 100 µL of the LAL-bound bead reagent produced in Production example 1. 50 µL of the dissolved solution was transferred to the glass vessel containing the endotoxin diluted water and measured using a stirring turbidimetric measurement device for endotoxin (EX-100, manufactured by Kowa Company Ltd.). Further, the limulus reagent was simultaneously dissolved in 100 µL of distilled water for injection in place of the LAL-bound bead reagent. 50 µL of the dissolved solution was transferred to the glass vessel containing the endotoxin diluted water and measured in the same manner.

When endotoxin is reacted with the limulus reagent, coagulins (insoluble proteins) are generated in the limulus reagent in the case of the sample not containing the LAL-bound bead. Thus, the blended liquid becomes turbid and the light transmittance is decreased. On the other hand, in the case of the blended liquid prepared by using the LAL-bound bead reagent, the reagent is made cloudy by the bead. However, in the first stage of the start of generation of coagulins, the coagulins are associated with the coagulins produced on the bead and the bead is aggregated. Accordingly, the turbidity is reduced and the light transmittance is once increased. A procedure of detecting an changing point where the light transmittance suddenly changed (parallel difference method) was used. The time when the changing point appeared was defined as the detection time of endotoxin.

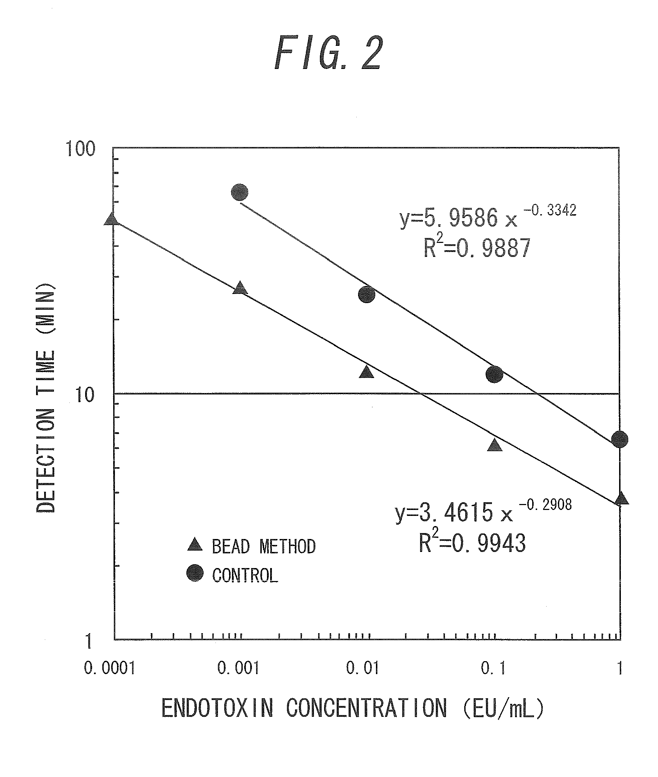

Fig. 2 is a graph obtained by plotting the concentration of endotoxin on the abscissa and the detection time on the ordinate by double logarithm. As shown in Fig. 2, it is found that when the LAL-bound bead reagent of the present invention is used, the measurement time can be significantly shortened while maintaining the measurement accuracy of the endotoxin concentration. As is clear from Fig. 2, when the LAL-bound bead reagent is not used, the detection time is extremely long in a region where the endotoxin concentration is low. Actually, a region which cannot be measured is large. Therefore, the measurement limit of the concentration of endotoxin can be expanded by using the LAL-bound bead reagent in this example.

### <Example 2> (Laser light scattering particle counting)

100 µL of endotoxin dilution series (prepared by diluting with distilled water for injection so as to be 2, 0.2, 0.02, 0.002, and 0.0002 EU/mL) was poured into the measuring glass vessel (φ7 mm) produced in Production example 3. Then, a limulus reagent (ES-II, manufactured by Wako Pure Chemical Industries, Ltd.) was dissolved in 100 µL of the LAL-bound bead reagent produced in Production example 1. The dissolved solution was transferred to the glass cuvette containing the endotoxin diluted water and measured using a laser light scattering particle counting device (EX-300, manufactured by Kowa Company Ltd.,).

Further, the limulus reagent was simultaneously dissolved in 100 µL of distilled water for injection in place of the LAL-bound bead reagent. The dissolved solution was transferred to the glass vessel containing the endotoxin diluted water and measured in the same manner. When endotoxin is reacted with the limulus reagent, coagulins (insoluble proteins) are generated in the limulus reagent in the case of the blended liquid not containing the LAL-bound bead. Thus, gel particles are generated in the sample. Since the particles scatter light, they are detected by EX-300 as gel particles.

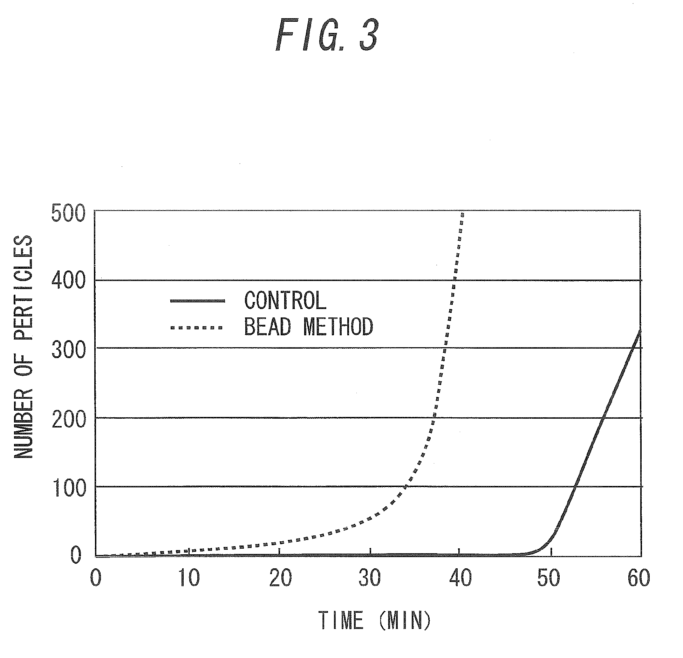

On the other hand, when the LAL-bound bead is contained in the blended liquid, the LAL-bound bead reagent produced in Production example 2 has little turbidity as compared with the LAL-bound bead reagent of Production example 1 . Even if the reagent is measured by EX-300, it is hardly detected as a light scattering body. However, when the reagent is acted on endotoxin and the reaction is proceeded, coagulins are produced in the sample and cross-linked to the LAL-bound bead. Thus, they are detected as gel particles. Fig. 3 shows a graph of a relation between the elapsed time after blending the LAL-bound bead reagent and endotoxin and the number of the detected particles.

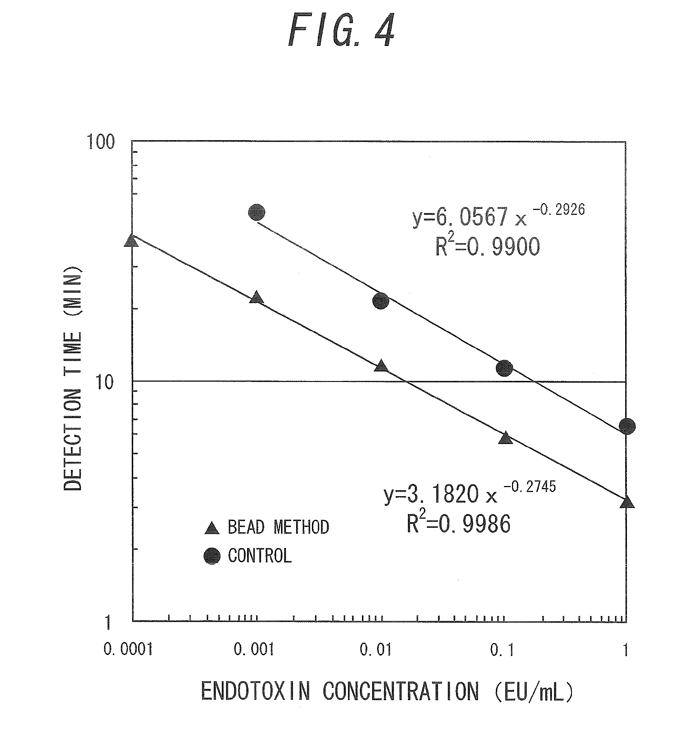

As shown in Fig. 3, when the LAL-bound bead is not present, it takes a long time until coagulins (about 5 nm) grow to a detectable size. However, when the LAL-bound bead is present, the associated beads 1 are detected as aggregated microparticles, without waiting for growth of coagulin particles alone to a sufficient size. Thus, a rise in the number of detected particles becomes earlier. Fig. 4 is a graph obtained by plotting the concentration of endotoxin on the abscissa and the detection time on the ordinate by double logarithm. As shown in Fig. 4, it is found that when the LAL-bound bead reagent of the present invention is used, the measurement time can be significantly shortened while maintaining the measurement accuracy of the endotoxin concentration. Note that in the present measurement, the detection of endotoxin is performed at the time when the total of the gel particles exceeds 20.

### <Example 3>

Subsequently, the case where titania particles are used as the bead 1 will be described. In this example, titania particles (Aeroxide P25, manufactured by Nippon Aerosil Co., Ltd.) were subjected to a dry heat treatment at 250°C for 3 hours, and the mixed endotoxin was completely inactivated by heat. Then, the resulting product was dispersed in distilled water for injection (manufactured by Otsuka Pharmaceutical Co., Ltd.) at a concentration of 10 mg/mL. 150 µL of the dispersed solution was added to a limulus reagent (HS-T, manufactured by Wako Pure Chemical Industries, Ltd.) to dissolve the reagent, followed by heating at 60°C for 20 minutes to allow proteins of the limulus reagent to be attached and immobilized onto the surfaces of particles. Further, the resulting product was diluted with distilled water for injection so as to be 3.0 mL. 2.0 mL of the diluted solution was dissolved in a limulus reagent (ES-II multi-test reagent, manufactured by Wako Pure Chemical Industries, Ltd.). 50 µL of the dissolved reagent was blended with 50 µL of an endotoxin dilution series solution in a measuring vessel, and the endotoxin agglutination reaction was measured by a stirring turbidimetric measurement device (EX-100, manufactured by manufactured by Kowa Company Ltd.).

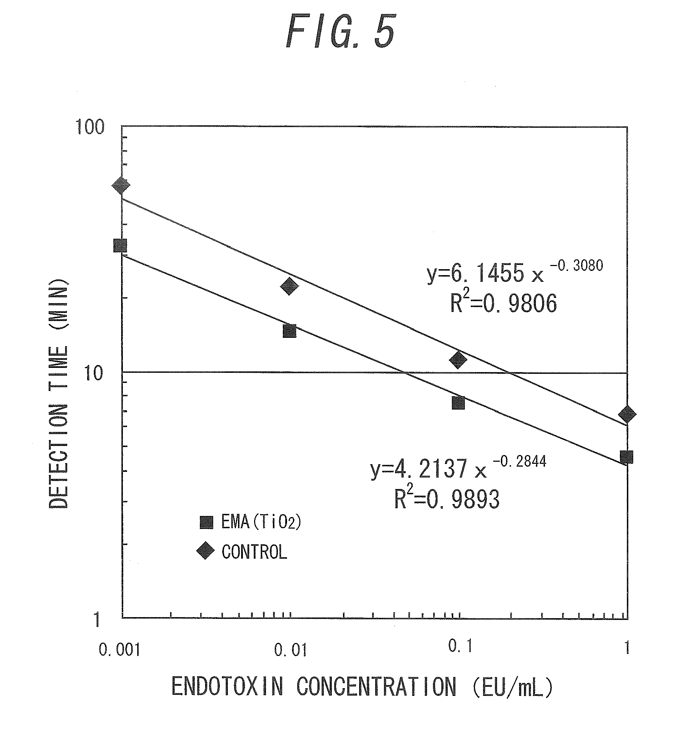

As the measurement result, almost the same phenomenon as the case where alumina was used as the bead 1 was observed. In other words, in an early stage of the aggregation reaction, the light transmittance was once increased by the association of the beads 1. Thereafter, the light transmittance was reduced. Fig. 5 shows a relation between the endotoxin concentration and the endotoxin detection time in both cases of using or not using the LAL-bound bead reagent in this example. As shown in Fig. 5, the logarithmic plot of the both axes was approximated to line. It was found that when the bead 1 of titania particle was used, the measurement time could be significantly shortened while maintaining the measurement accuracy of the endotoxin concentration.

### <Example 4>

Subsequently, as metal oxide microparticles to be used for the bead 1, alumina (spherical alumina ASFP-20, manufactured by DENKI KAGAKU KOGYO KABUSHIKI KAISHA), silica (Aerosil300, manufactured by Nippon Aerosil Co., Ltd.), titania (Aeroxide P25, manufactured by Nippon Aerosil Co., Ltd.), manganese oxide (IV) powder (manufactured by Kanto Kagaku) were used. As mineral microparticles to be used for the bead 1, hydroxyapatite (manufactured by Covalent Materials Corporation), kaoline (manufactured by Kanto Kagaku), and bentonite (manufactured by Kanto Kagaku) were used. An LAL-bound bead reagent was prepared using the bead 1 formed from these inorganic materials. Then, the effect of shortening the measurement time of endotoxin was examined.

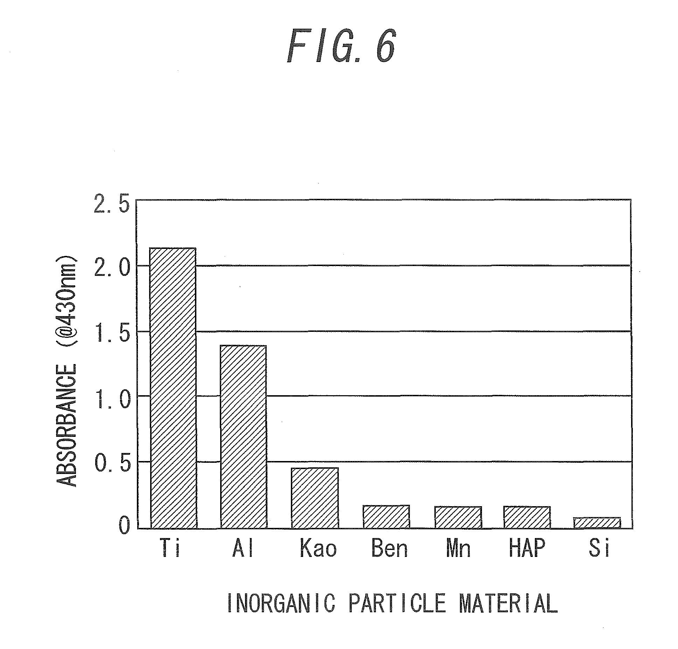

These beads 1 were subjected to a dry heat treatment at 250°C for 3 hours. The contaminated endotoxin was inactivated by heat and the resulting beads 1 were suspended in distilled water for injection so that the concentration of each of the beads 1 was 100 mg/mL. The particle suspension was diluted to 10 mg/mL and irradiated with light having a wavelength of 430 nm. Each absorbance was shown in Fig. 6.

As shown in Fig. 6, titania (Ti) exhibited the highest absorbance. The absorbances of alumina (Al) and kaoline (Kao) were reduced in this order. In the cases of each suspension of particles of bentonite (Ben), manganese oxide (Mn), hydroxyapatite (HAP), and silica (Si), the absorbance lower than that of the case of kaoline (Kao) was obtained. Subsequently, 50 µL of each suspension of particles was added to a limulus reagent (HS-T single test, manufactured by Wako Pure Chemical Industries, Ltd.) which had been previously dissolved in 50 µL of distilled water for injection, which was stirred with a vortex mixer. Each mixture was treated at 60°C for 30 minutes to allow the proteins in the limulus reagent to be immobilized on the particles.

Each suspension containing alumina and titania as a raw material was diluted with distilled water for injection so as to be 3.0 mL. Each suspension containing the other materials was diluted with distilled water for injection so as to be 1.5 mL. 50 µL of each bead liquid was blended with 50 µL of a limulus reagent (ES-II, manufactured by Wako Pure Chemical Industries, Ltd.) dissolved in an endotoxin diluted solution (0.02 EU/mL) . Then, the endotoxin agglutination reaction was examined by a stirring turbidimetric measurement device (EX-100, manufactured by manufactured by Kowa Company Ltd.).

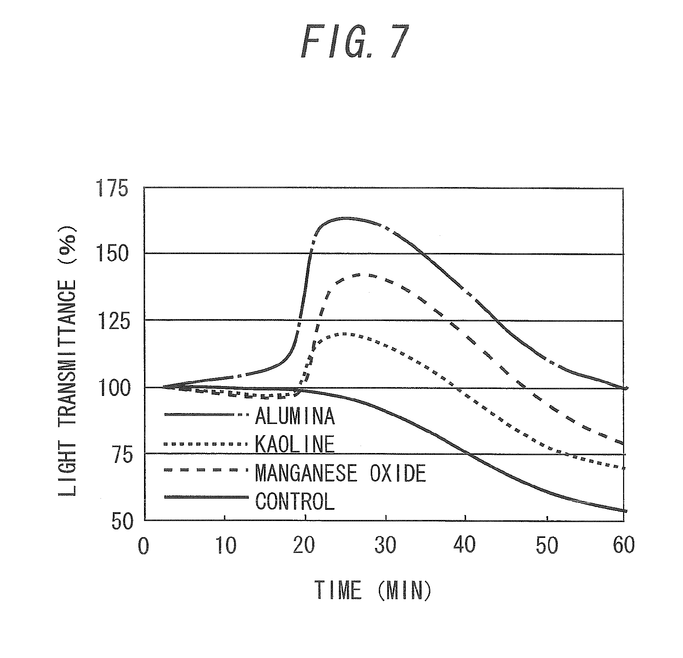

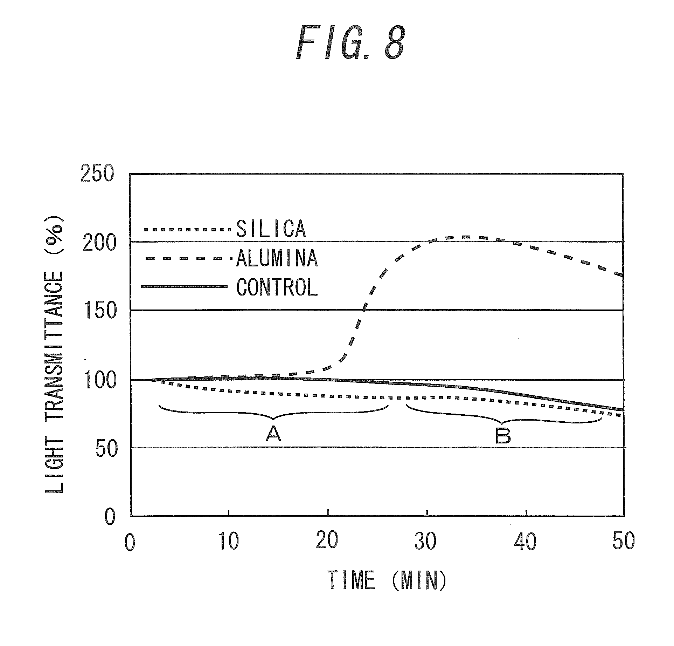

The measurement results are shown in Fig. 7. In Fig. 7, as for the inorganic particles examined this time, a phenomenon in which the light transmittance was increased by association of the beads 1 in an early stage of the agglutination reaction was obviously observed in kaoline and manganese oxide in addition to titania and alumina whose effects have been described. On the other hand, when silica and bentonite were used, a phase in which the light transmittance was increased in an early stage of the agglutination reaction was small. The effect by using the LAL-bound bead reagent was not so large. Fig. 8 shows the results at the time of using silica (a region A in Fig. 8 is a phase in which the light transmittance is decreased without reference to the endotoxin agglutination reaction, and a region B is a phase in which the light transmittance seems to be increased by aggregation of a number of particles). A cause that the rise of the light transmittance is decreased when using silica as described above is mainly attributed to that the absorbance per weight is low.

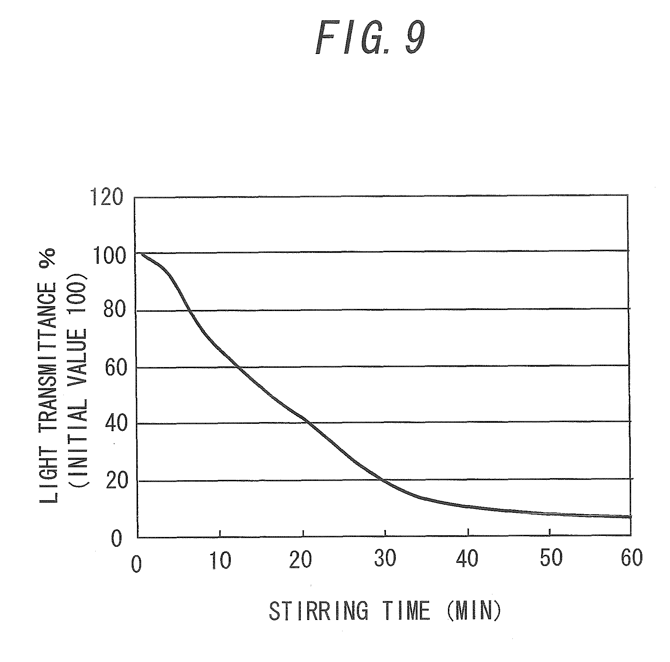

When hydroxyapatite was used as the bead 1, a phenomenon in which the light transmittance was decreased without reference to the aggregation reaction caused by the reaction of LAL and endotoxin was observed. The cause of the phenomenon in hydroxyapatite is considered as follows. Since hydroxyapatite has a low mechanical strength and is broken by stirring, the light transmittance is reduced by the bead 1 broken without reference to the agglutination reaction. Fig. 9 shows a graph of change of the light transmittance due to the breakdown of hydroxyapatite.

As described above, it is confirmed that, in the case of producing an LAL-bound bead reagent, it is preferable to use a material of the bead 1 which satisfies the following conditions: (1) adsorptive property to protein is high; (2) the absorbance per weight is high; and (3) a mechanical strength enough not to be broken by an external force such as stirring is provided. Further, it is confirmed that alumina, titania, kaoline, and manganese oxide are materials satisfy the above conditions and capable of being sufficiently used as the bead 1.

The inorganic material to be used for the microparticles of the present invention is not naturally limited to the exemplified ones. It is possible to use a material which satisfies the following conditions: (1) adsorptive property to protein is high; (2) the absorbance per weight is high; and (3) a mechanical strength enough not to be broken by an external force such as stirring is provided. Of course, a composite compound with the material composition satisfying the conditionsmaybeused. In addition, a material prepared by mixing a plurality of beads with different compositions may be used. For example, silica alumina, alumina titania, titania zirconia and the like can be used, and beads formed from alumina, titania, and silica may be mixed at an appropriate ratio for use.

Note that, in Production examples 1 and 2 in this embodiment, proteins are attached to the bead 1 without screening the proteins contained in LAL to prepare an LAL-bound bead reagent. However, as the above example, it is desired that when the LAL-bound bead reagent is mixed with the LAL reagent and blended with the sample containing endotoxin to allow the LAL-bound bead 10 to be aggregated, as many coagulogens as possible are attached onto the bead 1 forming the LAL-bound bead 10. Therefore, whenproducing the LAL-bound bead 10, proteins are not attached to the bead 1 without screening the proteins contained in LAL, but the purified coagulogens may be attached to the bead 1.

Note that, in the above examples, the prepared LAL-bound bead reagent corresponds to the reagent kit for measuring endotoxin in the present invention.

### Reference Signs List

- 1: Bead
- 2: Coagulogen
- 3: Coagulogen
- 10: LAL-bound bead

## Claims

1. A measurement method for a physiologically active substance of biological origin comprising:
allowing a predetermined protein contained in a limulus amoebocyte lysate to be attached to the surfaces of microparticles capable of being dispersed in a reagent;
blending the reagent containing the dispersed microparticles to which the protein is attached with a sample; and
detecting formation of a gel in a blended liquid of the reagent and the sample to detect the physiologically active substance of biological origin present in the sample or measuring the degree of formation of the gel to measure the concentration of the physiologically active substance; **characterized in that**:
the microparticles are formed from an inorganic material.

2. The measurement method for a physiologically active substance of biological origin according to claim 1, wherein the microparticles are formed from a metal oxide or a mineral.

3. The measurement method for a physiologically active substance of biological origin according to claim 1, wherein the microparticles are formed from any of materials of alumina, titania, kaoline, and manganese oxide.

4. The measurement method for a physiologically active substance of biological origin according to any one of claims 1 to 3, wherein the predetermined protein is a coagulogen purified from the limulus amoebocyte lysate.

5. The measurement method for a physiologically active substance of biological origin according to any one of claims 1 to 4, further comprising:
mixing the reagent containing the dispersed microparticles to which the predetermined protein is attached with the limulus amoebocyte lysate; and
blending the reagent and the sample simultaneously with or after the mixing.

6. A reagent kit for measuring a physiologically active substance of biological origin which is prepared by allowing a predetermined protein contained in a limulus amoebocyte lysate to be attached to the surfaces of microparticles capable of being dispersed in a reagent; wherein the microparticles are formed from an inorganic material.

7. The reagent kit for measuring a physiologically active substance of biological origin according to claim 6, wherein the microparticles are formed from a metal oxide or a mineral.

8. The reagent kit for measuring a physiologically active substance of biological origin according to claim 6, wherein the microparticles are formed from any of materials of alumina, titania, kaoline, and manganese oxide.

9. The reagent kit for measuring a physiologically active substance of biological origin according to any one of claims 6 to 8, wherein the predetermined protein is a coagulogen purified from limulus amoebocyte lysate.
